# EUROPEAN PATENT APPLICATION

(11) **EP 2 486 979 A2**
(43) Date of publication of application: **15.08.2012**
(21) Application number: 12153049.7
(22) Date of filing: 30.01.2012
(51) Int. Cl.: B01L 3/00

(54) **Microfluidic device**

(30) Priority: 09.02.2011 KR 20110011513
(71) Applicant: Samsung Electronics Co., Ltd., Suwon-si, Gyeonggi-do 442-742 (KR)
(72) Inventor: Lee, Jong Gun, Gyeonggi-do (KR); Kim, Na Hui, Gyeonggi-do (KR); Lee, Young Goun, Seoul (KR)
(74) Representative: Grootscholten, Johannes A.M.

(57) **Abstract**

A microfluidic device (10) to meter an accurate amount of fluid is provided. The microfluidic device (10) includes a chamber (110) configured to accommodate a fluid therein, an inlet channel (120) connected to the chamber (110) to supply the fluid into the chamber (110), and an outlet channel (130) connected to the chamber (110) and provided separately from the inlet channel (120). The outlet channel (130) is configured such that after the chamber (110) is filled with a predetermined amount of the fluid introduced through the inlet channel (120), any additional amount of the fluid introduced into the chamber (110) in excess of the predetermined amount exits the chamber (110) through the outlet channel (130).

## Description

### BACKGROUND

### 1. Field

Apparatuses and methods consistent with exemplary embodiments relate to a microfluidic device having a metering structure to meter an accurate amount of fluid.

### 2. Description of the Related Art

A lab-on-a-chip refers to a device in which microfluidic structures are arranged in a chip-shaped substrate to implement multiple steps of processing and operations, in order to implement tests including biochemical reactions on a small chip.

Transferring a fluid in microfluidic structures may require a drive pressure, such as capillary pressure or pressure produced by a separate pump. Recently, disc-type microfluidic devices, in which microfluidic structures are arranged on a disc-shaped platform to enable centrifugal movement of fluid, have been suggested to implement a series of operations. Such a disc-type microfluidic device is called a lab compact disc (CD), lab-on a disc or digital bio disc (DBD).

Generally, in the related art, a disc-type microfluidic device includes chambers to confine a fluid therein, channels for fluid flow, and valves to control flow of a fluid, all of which may be combined in various ways.

A microfluidic device may serve as a sample analysis device to test a sample, such as blood, saliva, urine, etc. A reagent, which is reactable with a specific ingredient of the sample may be accommodated in the microfluidic device. Testing the sample may be performed by injecting a sample into the microfluidic device and detecting results of reaction between the sample and the reagent.

To obtain reliable sample test results, it may be necessary to accurately meter a preset amount of sample that will react with a reagent.

During fluid metering, however, a smaller amount of fluid than a preset amount may be measured due to the nature of the fluid, such as viscosity, superficial energy, etc., or external factors, such as surrounding temperature, rotational speed of the microfluidic device, etc.

### SUMMARY

Exemplary embodiments provide a microfluidic device to meter an accurate amount of fluid.

In accordance with an aspect of an exemplary embodiment, there is provided a microfluidic device including a chamber configured to accommodate a fluid therein, an inlet channel connected to the chamber and configured to supply the fluid into the chamber, and an outlet channel connected to the chamber and provided separately from the inlet channel, wherein the outlet channel is configured such that after the chamber is filled with a predetermined amount of the fluid introduced through the inlet channel, any additional amount of the fluid introduced into the chamber in excess of the predetermined amount exits the chamber through the outlet channel.

The inlet channel may include a first inlet channel to guide the fluid toward the chamber and a second inlet channel to connect the first inlet channel to the chamber.

The outlet channel may include a first outlet channel to guide the fluid emerging from the chamber, and a second outlet channel to connect the first outlet channel to the chamber.

The chamber may include a metering chamber configured to accommodate and discharge a predetermined amount of fluid.

The microfluidic device may further include a discharge channel to allow the fluid accommodated in the chamber to be discharged to the outside of the chamber.

The microfluidic device may further include a valve to open and/or close the discharge channel.

Centrifugal force may be applied to the inlet channel and/or the outlet channel to enable movement of the fluid. In an exemplary embodiment, the centrifugal force applied to the outlet channel may be greater than the centrifugal force applied to the inlet channel.

In accordance with an aspect of another exemplary embodiment, there is provided a microfluidic device including a platform which includes a chamber configured to accommodate a fluid therein, an inlet channel configured to supply the fluid into the chamber, and an outlet channel provided separately from the inlet channel, wherein the outlet channel is configured such that after the chamber is filled with a predetermined amount of the fluid introduced through the inlet channel, any additional amount of the fluid introduced into the chamber in excess of the predetermined amount exits the chamber through the outlet channel.

The chamber may include a metering chamber configured to accommodate and discharge a predetermined amount of fluid.

The chamber may be located at the outer side of the inlet channel in a radial direction of the platform.

The chamber may be located at the outer side of the outlet channel in a radial direction of the platform.

The distance from the rotational center of the platform to the outlet channel may be greater than the distance from the rotational center of the platform to the inlet channel such that the centrifugal force applied to the outlet channel is greater than the centrifugal force applied to the inlet channel during rotation of the platform.

The inlet channel may include a first inlet channel to guide the fluid toward the chamber and a second inlet channel to connect the first inlet channel to the chamber.

The outlet channel may include a first outlet channel to guide the fluid emerging from the chamber, and a second outlet channel to connect the first outlet channel to the chamber.

The second outlet channel may be offset by a predetermined angle from a radial direction of the platform to a movement direction of the fluid in the first outlet channel, to ensure that the fluid emerging from the chamber moves into the first outlet channel.

In accordance with an aspect of another exemplary embodiment, there is provided a microfluidic device including a platform, a chamber configured to accommodate a fluid therein, an inlet channel connected to the chamber to supply the fluid into the chamber, an outlet channel connected to the platform separately from the inlet channel, a discharge channel connected to the chamber to discharge the fluid accommodated in the chamber, and a valve disposed at the discharge channel to open or close the discharge channel, wherein after the chamber is filled with a predetermined amount of the fluid introduced through the inlet channel, any additional amount of the fluid introduced into the chamber in excess of the predetermined amount exits the chamber through the outlet channel.

In accordance with an aspect of another exemplary embodiment, there is provided a method of filling a chamber of a microfluidic device with a predetermined amount of fluid, the method including supplying a fluid into the chamber through an inlet channel of the microfluidic device connected to the chamber, after the chamber is filled with a predetermined amount of the fluid through the inlet channel, allowing any additional amount of the fluid introduced to the chamber in excess of the predetermined amount to exit the chamber through an outlet channel of the microfluidic device that is connected to the chamber separately from the inlet channel, and stopping the outflow of fluid through the outlet channel if the supply of the additional or excess fluid is stopped, so as to allow the predetermined amount of the fluid to remain in the chamber.

### BRIEF DESCRIPTION OF THE DRAWINGS

The above and/or other aspects will become apparent and more readily appreciated from the following description of exemplary embodiments, taken in conjunction with the accompanying drawings of which:
FIG. 1 is a perspective view illustrating the external appearance of a microfluidic device according to an exemplary embodiment;
FIG. 2 is a plan view illustrating the internal configuration of a microfluidic device according to an exemplary embodiment;
FIG. 3 is an enlarged perspective view illustrating a metering chamber included in a microfluidic device according to an exemplary embodiment;
FIG. 4 is an enlarged plan view of the metering chamber; and
FIG. 5 is a diagram of a sample testing apparatus using a microfluidic device according to an exemplary embodiment.

### DETAILED DESCRIPTION

Reference will now be made in detail to an exemplary embodiment, examples of which are illustrated in the accompanying drawings, wherein like reference numerals refer to like elements throughout.

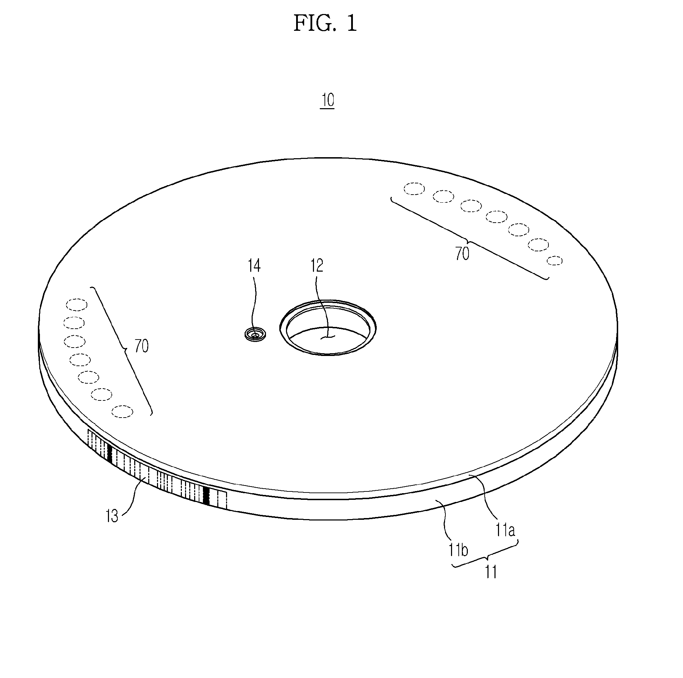

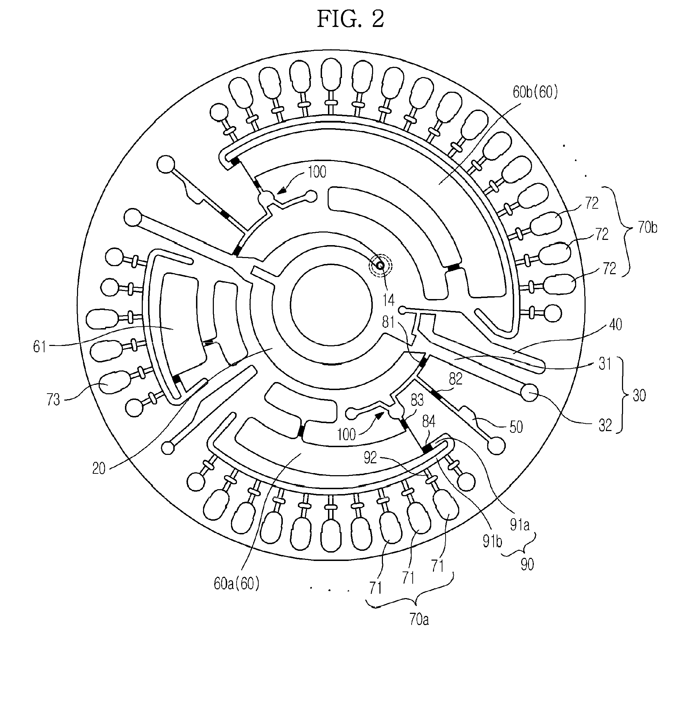

FIG. 1 is a perspective view illustrating the external appearance of a microfluidic device according to an exemplary embodiment, and FIG. 2 is a plan view illustrating the internal configuration of the microfluidic device according to an exemplary embodiment.

As illustrated in FIGS. 1 and 2, the microfluidic device 10 includes a disc-shaped platform 11, a plurality of chambers for fluid accommodation and a plurality of channels for fluid flow, which are defined in the platform 11, and a barcode 13 provided at a lateral surface of the platform 11.

The platform 11 is rotatable about a center axis 12 thereof. The chambers and channels provided in the platform 11 may perform movement, centrifugal separation and/or mixing of a sample under the influence of centrifugal force generated by rotation of the platform 11.

In an exemplary embodiment, the platform 11 may be formed of plastics which are easy to mold and have biologically inert superficial properties, such as acryl, polydimethylsiloxane (PDMS). However, the platform 11 is not limited to this embodiment and may be formed of other materials having chemical and biological stability, excellent optical transparency and mechanical workability.

The platform 11 may be constructed from multilayered plates. The platform 11 may provide spaces and passages by forming intaglio structures corresponding to chambers, channels, etc., at interfaces of contact plates and bonding the plates to each other.

For example, the platform 11 may consist of a first substrate 11a and a second substrate 11b attached to the first substrate 11a, or may include first and second plates 11a and 11b with a partition (not shown), which defines chambers for fluid accommodation and channels for fluid flow, interposed therebetween. Additionally, the platform 11 may have various other configurations. The first substrate 11a and the second substrate 11b may be formed of thermoplastic resins.

Bonding of the first substrate 11a and the second substrate 11b to each other may be accomplished by various methods, such as adhesion, an adhesive, double-sided tape, ultrasonic fusion, laser welding, etc.

Hereinafter, exemplary microfluidic structures arranged within the platform 11 for use in a sample test will be described.

A sample may be a blend of a fluid and a particulate substance having a greater density than that of the fluid. For example, the sample may include a bodily sample, such as blood, salvia, urine and the like.

A sample chamber 20 may be defined in a radially inward location of the platform 11 to accommodate a predetermined amount of sample. At the top of the sample chamber 20, there may be provided a sample injection port 14 through which a sample is introduced into the sample chamber 20.

In certain circumstances, it may be necessary to test only the fluid, rather than the entire sample, which may include both the fluid and the particulate substance. Thus, a sample separating chamber 30 may be provided radially at the outer side of the sample chamber 20 to centrifugally separate the sample via rotation of the platform 11.

In an exemplary embodiment, an extra sample chamber 40 may be provided at one side of the sample separating chamber 30. Thus, if excess sample is injected into the sample chamber 20, only a predetermined amount of the sample required for the test remains in the sample separating chamber 30 and the excess sample is accommodated in the extra sample chamber 40.

The sample separating chamber 30 includes a channel-shaped supernatant collecting region 31 radially outwardly extending from the sample chamber 20 and a sediment collecting region 32 radially at the outer side of the supernatant collecting region 31 to provide a space in which sediment having a relatively larger specific gravity may be accommodated. If the sample is blood, blood cells are collected in the sediment collecting region 32 and serum having a lower specific gravity than the blood cells is collected in the supernatant collecting region 31 during rotation of the platform 11. Thus, the blood is divided into blood cells and serum in the sample separating chamber 30, and the serum may be used for testing.

Then, the sample is introduced into a metering structure 100 so as to be metered to a preset amount that is required for the test. Although FIG. 2 illustrates the metering structure 100 as being connected to the sample separating chamber 30, the microfluidic device 10 may lack the sample separating chamber 30 and therefore, the metering structure 100 may be directly connected to the sample chamber 20.

A residual sample removal chamber 50 may be provided around the metering structure 100 to remove the excess sample after the sample has been metered by the metering structure 100.

At least one dilution chamber 60 may be connected to the metering structure 100 to receive a preset amount of sample. The at least one dilution chamber 60 may include a plurality of dilution chambers (e.g., 60a and 60b) in which different amounts of dilution buffer are stored respectively. The volume of the plurality of dilution chambers 60 may vary according to a required volume of dilution buffer. In an exemplary embodiment, the microfluidic device 10 includes first and second dilution chambers 60a and 60b in which different volumes of dilution buffer are accommodated respectively to exhibit different dilution ratios.

Additionally, a dilution chamber 61 may be provided within the platform such that no sample is supplied from the sample separating chamber 30 to the dilution chamber 61. The dilution chamber 61, to which no sample is supplied, functions to obtain a standard value (e.g., a positive and/or negative control) and is configured to accommodate a dilution buffer. A plurality of chambers 73 may be provided at the outer side of the dilution chamber 61 to which no sample is supplied. The chambers 73 may be empty or may be filled with distilled water and function to obtain a detection standard value.

A distribution channel 90 is connected to an exit of the dilution chamber 60. The distribution channel 90 includes a first section 91a extending from the exit of the dilution chamber 60 in a radial outward direction of the platform 11, and a second section 91b circumferentially extending from an outer end of the first section 91a. A distal end of the second section 91b may be connected to a vent (not shown). The vent (not shown) may be positioned to prevent leakage of the sample when the sample is transferred using centrifugal force from the dilution chamber 60 to the distribution channel 90. The distribution channel 90 may achieve a constant fluid resistance from a front end thereof connected to the exit of the dilution chamber 60 to a rear end thereof connected to the vent (not shown), i.e., throughout the entire section including the first section 91a and the second section 91b. To achieve the constant fluid resistance, the distribution channel 90 may have a constant cross sectional area. Thereby, rapid and effective distribution of the sample may be accomplished by excluding possible fluid movement resistance during distribution of the fluid to the maximum extent possible.

Reaction chamber groups 70a and 70b may be arranged at the outer side of the first and second dilution chambers 60a and 60b, respectively. Specifically, a first reaction chamber group 70a is provided at the outer side of the corresponding first dilution chamber 60a, and a second reaction chamber group 70b is provided at the outer side of the corresponding second dilution chamber 60b.

Each reaction chamber group 70a or 70b includes at least one reaction chamber 71 or 72. The reaction chamber 71 or 72 is connected to the corresponding dilution chamber 60 through the distribution channel 90 that distributes the dilution buffer. In the simplest configuration, the reaction chamber group 70a or 70b may include a single reaction chamber.

The reaction chamber 71 or 72 may be a hermetic chamber. As used herein, the term "hermetic" means that each reaction chamber 71 or 72 has no vent for gas exhaust. Various kinds or concentrations of reagents, which cause optically detectable reactions with a sample dilution buffer distributed through the distribution channel 90, may be previously introduced into the plurality of reaction chambers 71 and 72. The reagents may be accommodated in a solid phase within the reaction chambers 71 and 72. Examples of optically detectable reactions include, but are not limited to, variation in fluorescence and/or optical density. However, the purposes of the reaction chambers 71 and 72 are not limited to the above.

In an exemplary embodiment, the plurality of reaction chambers 71 or 72 may store reagents suitable for reactions with the sample dilution buffer at the same dilution ratio.

For example, the first reaction chamber group 70a may store reagents, such as triglycerides (TRIG), total cholesterol (Chol), glucose (GLU), urea nitrogen (BUN), etc., which react under a dilution ratio (dilution buffer/sample) of 100. Likewise, the second reaction chamber group 70b may store reagents, such as direct trilirubin (DBIL), total bilirubin (TBIL), gamma glutamyl transferase (GGT), etc., which react under a dilution ratio (dilution buffer/sample) of 20.

In other words, if a sample dilution buffer, which is supplied from the second dilution chamber 60b to the plurality of reaction chambers 72 of the corresponding second reaction chamber group 70b, has a different dilution ratio than the first reaction chamber group 70a, the reaction chambers 71 and 72 of the reaction chamber groups 70a and 70b may respectively store reagents suitable for specific dilution ratios of the sample.

The reaction chambers 71 and 72 may have the same capacity, but are not limited as such. In embodiments in which different capacities of sample dilution buffer or reagent are required according to test items, the capacities of the reaction chambers 71 and 72 may differ from each other.

The plurality of reaction chambers 71 and 72 may be chambers provided with vents and injection ports.

The plurality of reaction chambers 71 and 72 are individually connected to the second section 91b of the distribution channel 90 through an entrance channel 92.

In an exemplary embodiment, channels connecting the respective chambers to each other may be provided with valves, e.g., 81, 82, 83 and 84. The valves 81, 82, 83 and 84 may include a first valve 81 provided at an exit side of the sample separating chamber 30, a second valve 82 provided at an entrance side of the residual sample removal chamber 50, a third valve 83 provided at an exit side of the metering structure 100, and a fourth valve 84 disposed at an exit side of the dilution chamber 60 so as to open or close the distribution channel 90. The valves 81, 82, 83 and 84 used herein may be normally closed valves.

The respective valves 81, 82, 83 and 84 may be various kinds of valves, such as valves that are passively opened if a predetermined pressure or more is applied (like capillary valves), or valves that are actively operated upon receiving power or energy from the outside in response to actuation signals. In one exemplary embodiment, the microfluidic device 10 employs one or more phase transition valves that are operated by absorbing energy from the outside.

Each valve is positioned between upper and lower plates of the platform 11 and has a three-dimensional or planar shape as described above. The valve serves to block the flow of fluid and is melted at a high temperature to move into an adjacent clearance so as to open the channel.

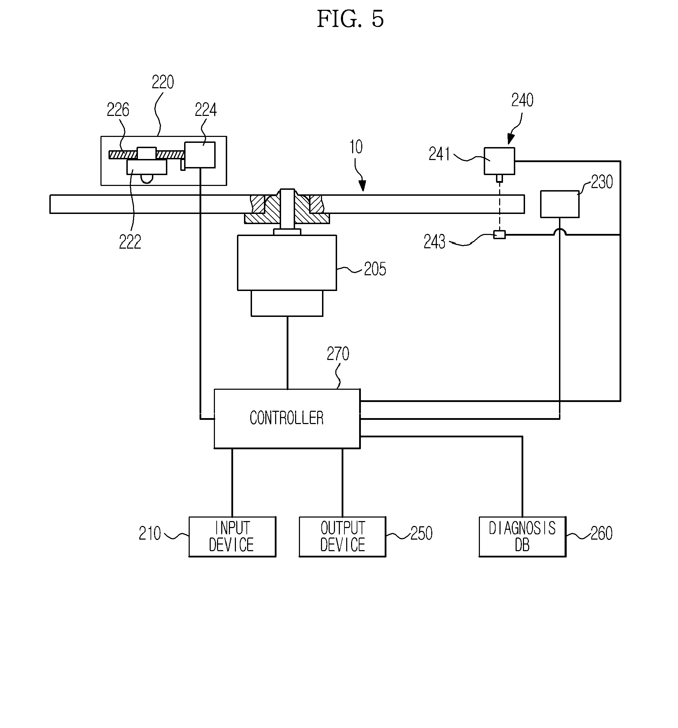

To apply heat to the valves 81, 82, 83 and 84, an external energy source (222, see FIG. 5) to emit light is movably disposed at the outside of the platform 11. The external energy source 222 may irradiate light to sites where the valves 81, 82, 83 and 84 are located.

Thus, the external energy source 222 is moved to above any one of the valves 81, 82, 83 and 84 that is necessary to be opened according to the test protocol of the disc-type microfluidic device 10, and irradiates light (or energy) onto the corresponding valve 81, 82, 83 or 84 for valve opening.

The valves 81, 82, 83 and 84 may be formed of a phase transition substance, with heat emitting particles, which absorb electromagnetic waves and then generate heat, distributed throughout the phase transition substance. In one exemplary embodiment, the valve material may include a phase transition material which is solid at room temperature, such as wax. The wax may be solid at room temperature and become liquid when it is heated. Exemplary waxes, include but are not limited to, paraffin wax, microcrystalline wax, synthetic wax, natural wax or the like.

The heat emitting particles may be sized to be freely movable within a channel having a width of several hundred to thousands of micrometers. If light (e.g., a laser) is irradiated onto the heat emitting particles, the temperature of the heat emitting particles rapidly increases. In order to exhibit the foregoing properties, each of the heat emitting particles may be formed from a metallic core and a hydrophobic shell. For example, the heat emitting particle may include an iron (Fe) core and a shell consisting of multiple surfactants bonded to the Fe core to enclose the Fe core. As an example of the heat emitting particles, commercially available ones distributed in a carrier oil may be adopted.

The heat emitting particles are not particularly limited to polymer particles, as provided herein for illustrative purposes. In another exemplary embodiment, the heat emitting particles may be in a quantum dot or magnetic bead form. In another exemplary embodiment, the heat emitting particles may be micro-metal oxides such as A1203, TiO2, Ta2O3, Fe2O3, Fe3O4 or HfO2.

In another exemplary embodiment, the heat emitting particles may be stored in a dispersion state in a carrier oil. The carrier oil may also be hydrophobic to allow the heat emitting particles having the hydrophobic surface structure to be uniformly dispersed in the carrier oil. Thus, a channel (e.g., 140) may be closed by forming a uniform dispersion of the melted transition material and the carrier oil containing the micro-heating particles, and introducing the mixture into the channel 140.

If the heat emitting particles convert absorbed energy into thermal energy, the particles rapidly heat up and transfer the thermal energy to the surrounding area. Thus, the wax is melted by the thermal energy, which results in collapse of the valve and opening of the channel. The wax may have a moderate melting point. If the melting point is excessively high, the period of time from energy irradiation to valve opening is excessively increased, which makes it difficult to accurately control valve opening time. On the contrary, if the melting point is excessively low, the wax may be partially melted in a state in which no light is irradiated, thereby causing fluid leakage.

In another exemplary embodiment, the phase transition substance may be a gel or thermoplastic resin. Gels may be selected from polyacrylamide, polyacrylates, polymethacrylates, polyvinylamides, or the like. Thermoplastic resins may be selected from cyclic olefin copolymer (COC), polymethylmethacrylate (PMMA), polycarbonate (PC), polystyrene (PS), polyoxymethylene acetal polymer (POM), perfluoroalkoxy copolymer (PFA), polyvinyl chloride (PVC), polypropylene (PP), polyethylene terephthalate (PET), polyetheretherketone (PEEK), polyamide (PA), polysulphone (PSU), polyvinylidene fluoride (PVDF) or the like.

In one exemplary embodiment, a barcode 13 may be provided at the lateral surface of the platform 11. The barcode 13 may store a variety of information as necessary, including, but not limited to, a date of manufacture, expiration date, etc.

The barcode 13 may be selected from various types of barcodes. For example, the barcode 13 may be of a one-dimensional barcode type, or of a two-dimensional barcode type (e.g., a matrix code) to store a great quantity of information.

In certain exemplary embodiments, the barcode 13 may be replaced by a hologram, radio frequency identification (RFID) tag, or memory chip, used to store information therein. In the case where the barcode 13 is replaced by a storage medium, such as, e.g., a memory chip to enable reading and writing of information, it may be possible to store identification information and other information related to sample testing results, patient information, blood collecting/testing date and time, and whether or not a test has been executed.

A sample reacts with a reagent after being diluted in the dilution chamber 60. If the amount of the reagent is greater or less than a preset amount (i.e., the amount required for the particular test), the results of reaction with the reagent may exhibit low reliability.

Thus, the microfluidic device 10 includes a metering structure 100 to accurately meter a preset amount of sample for the particular test to be performed.

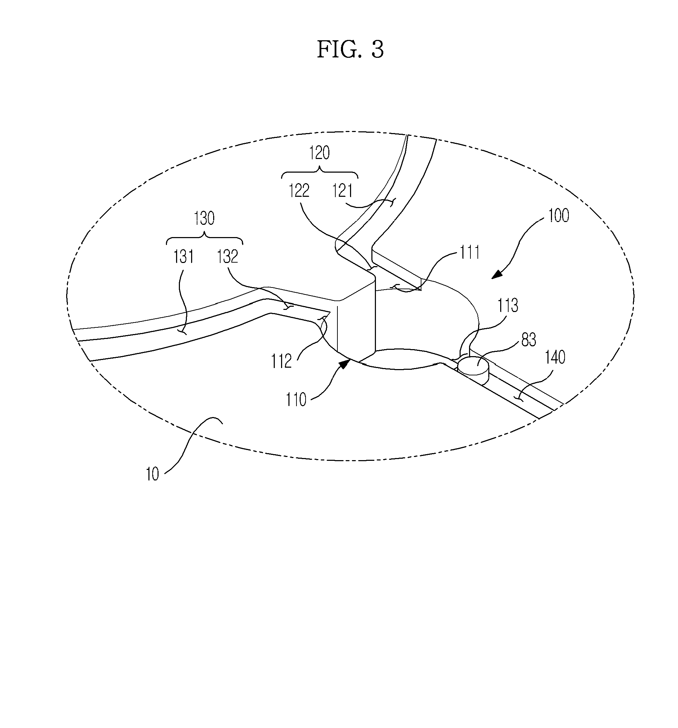

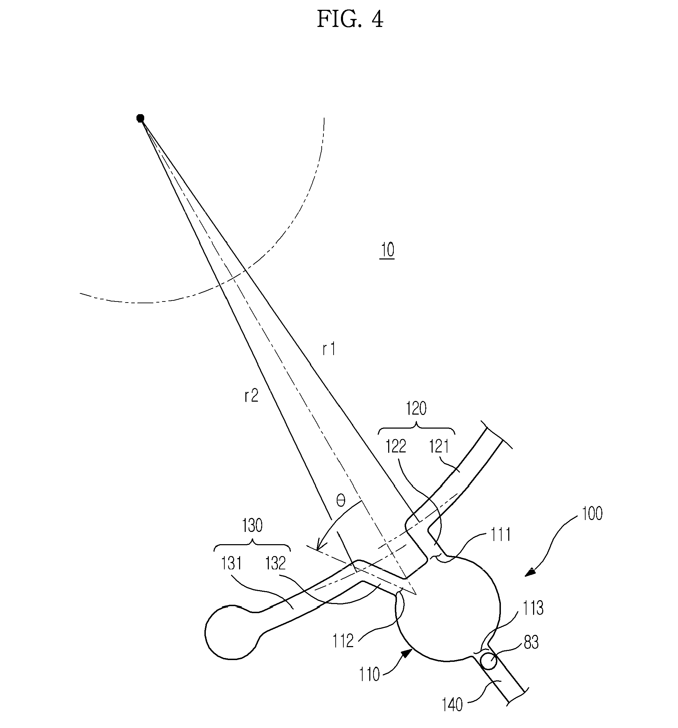

FIG. 3 is an enlarged perspective view illustrating a metering chamber included in a microfluidic device according to an exemplary embodiment, and FIG. 4 is an enlarged plan view of the metering chamber.

As illustrated in FIGS. 3 and 4, the metering structure 100 includes a metering chamber 110 in which a fluid is accommodated, an inlet channel 120 through which a fluid is supplied into the metering chamber 110, an outlet channel 130 through which a fluid exits the metering chamber 110, and a discharge channel 140 to discharge the fluid accommodated in the metering chamber 110.

The metering chamber 110 is configured to accommodate a predetermined amount of fluid. The metering chamber 110 has an inlet 111 for fluid inflow and an outlet 112 for fluid outflow. Once the metering chamber 110 has been filled with a preset amount of fluid, any additionally introduced fluid exits the metering chamber 110 through the outlet 112. The metering chamber 110 further has a discharge port 113 to discharge the fluid accommodated in the metering chamber 110 to the outside. While the metering chamber 110 is being filled with a fluid, i.e., while a fluid is passing through the inlet 111 and the outlet 112, the discharge port 113 remains in a closed state to prevent the fluid in the metering chamber 110 from being discharged therethrough.

In one exemplary embodiment, a fluid is introduced into the metering chamber 110 through the inlet 111 by centrifugal force generated during rotation of the platform 11. The inlet 111 may be located closer to the innermost region than the outermost region of the metering chamber 110 in a radial direction about the rotational center of the platform 11. This may prevent backflow of the fluid through the inlet 111 before the metering chamber 110 is full of the fluid and also, may prevent leakage of a part of the fluid through the inlet 111 when the fluid accommodated in the metering chamber 110 is discharged through the discharge port 113 because the metering chamber 110 is filled starting from the outermost region thereof by centrifugal force.

In another exemplary embodiment, the outlet 112 may be located closer to the innermost region than the outermost region of the metering chamber 110 in a radial direction about the rotational center of the platform 11. Positioning of the outlet 112 close to the innermost region of the metering chamber 110 allows a fluid to exit the metering chamber 110 through the outlet 112 after the metering chamber 110 is full of the fluid.

Once the metering chamber 110 is full of a preset amount of fluid, the discharge port 113 is opened, causing the fluid accommodated in the metering chamber 110 to be discharged from the metering chamber 110 through the discharge port 113 by centrifugal force. To ensure that all the fluid is discharged through the discharge port 113, the discharge port 113 may be located at a region of the metering chamber 110 where the largest centrifugal force acts, i.e., the farthest region of the metering chamber 110 from the rotational center of the platform 11.

The inlet channel 120 is connected at one end thereof to the inlet 111 of the metering chamber 110 so as to supply a fluid into the metering chamber 110. The other end of the inlet channel 120 may be connected to the sample separating chamber 30 illustrated in FIG. 2. During rotation of the platform 11, a fluid moves along the inlet channel 120 by centrifugal force, being introduced into the metering chamber 110. The inlet channel 120 may extend circumferentially about the rotational center of the platform 11. To allow the fluid to move to the metering chamber 110 by centrifugal force, at least a part of the inlet channel 120 close to the metering chamber 110 may extend radially from the rotational center of the platform 11.

Thus, the inlet channel 120 may include a first inlet channel 121, which extends in the circumferential direction of the platform 11 so as to be spaced apart from the metering chamber 110 in the radial direction of the platform 11, and a second inlet channel 122 which connects the first inlet channel 121 and the inlet 111 of the metering chamber 110 to each other. The second inlet channel 122 may extend in the radial direction of the platform 11 to guide the fluid in the radial direction of the platform 11. With provision of the second inlet channel 122, the inlet 111 of the metering chamber 110 may be positioned at the innermost region of the metering chamber 110 in the radial direction about the rotational center of the platform 11.

One end of the outlet channel 130 is connected to the outlet 112 of the metering chamber 110. If additional or excess fluid is introduced through the inlet channel 120 after the metering chamber 110 is full of a preset amount of fluid, the same amount of fluid as the additional or excess fluid exits the metering chamber 110. The outlet channel 130 may extend circumferentially about the rotational center of the platform 11. To allow the fluid to exit the metering chamber 110 by centrifugal force, at least a part of the outlet channel 130 close to the metering chamber 110 may extend radially from the rotational center of the platform 11.

Thus, the outlet channel 130 may include a first outlet channel 131, which extends in the circumferential direction of the platform 11 so as to be spaced apart from the metering chamber 110 in the radial direction of the platform 11, and a second outlet channel 132 which connects the first outlet channel 131 and the outlet 112 of the metering chamber 110 to each other. The second outlet channel 132 guides the fluid radially inward of the platform 11 and allows the outlet 112 of the metering chamber 110 to be positioned at the innermost region of the metering chamber 110 in the radial direction about the rotational center of the platform 11.

Since the second outlet channel 132 is configured to guide the fluid in an opposite direction of centrifugal force applied to the fluid, the fluid may need to overcome the centrifugal force applied to the fluid in order to move along the second outlet channel 132. If fluid is continuously introduced through the inlet channel 120 after the metering chamber 110 is full of the fluid, the internal pressure of the metering chamber 110 rises. Thus, the fluid may be pushed into the outlet channel 130 by the pressure difference between the metering chamber 110 and the outlet channel 130.

To ensure movement of the fluid along the second outlet channel 132, the second outlet channel 132 is offset by a predetermined angle θ from the radial direction of the platform 11 to a movement direction of the fluid in the first outlet channel 132.

The discharge channel 140 is connected to the discharge port 113 of the metering chamber 110 to extend radially outward from the rotational center of the platform 11. The discharge channel 140 is provided with a third valve 83, as shown in FIG. 2. The third valve 83 closes the discharge channel 140 while the fluid is being supplied into the metering chamber 110. If the discharge channel 140 is opened after the metering chamber 110 is full of a preset amount of fluid, the fluid accommodated in the metering chamber 110 is discharged from the metering chamber 110 through the discharge channel 140 by centrifugal force.

Accordingly, the metering chamber 110 is connected to the inlet channel 120 and the outlet channel 130 to define a flow path along the inlet channel 120 and the outlet channel 130 in a state in which the discharge channel 140 is closed by the third valve 83. That is, the metering chamber 110 constitutes a part of the fluid flow path.

After the metering chamber 110 is full of fluid, any amount of additional or excess fluid introduced into the metering chamber 110 through the inlet channel 120 exits the metering chamber 110 through the outlet channel 130. To ensure that the fluid efficiently exits the metering chamber 110 through the outlet channel 130, the magnitude of centrifugal force applied to the fluid moving along the outlet channel 130 during rotation of the platform 11 may be greater than centrifugal force applied to the fluid moving along the inlet channel 120. In particular, as the first inlet channel 121 and the first outlet channel 131 circumferentially extend about the rotational center of the platform 11, centrifugal force applied to the fluid moving along the first outlet channel 131 may be greater than centrifugal force applied to the fluid moving along the first inlet channel 121. This may be accomplished where a distance r2 from the rotational center of the platform 11 to the first outlet channel 131 is greater than a distance r1 from the rotational center of the platform 11 to the first inlet channel 121.

Although the metering chamber 110 has been described above, such description is applicable to all chambers or structures designed to accommodate a preset amount of fluid.

FIG. 5 is a diagram of a sample testing apparatus using a microfluidic device according to an exemplary embodiment.

The sample testing apparatus includes a spindle motor 205 to rotate the microfluidic device 10, a data reading device 230, a valve opening device 220, an inspection device 240, an input device 210, an output device 250, a diagnosis DB 260, and a controller 270 to control the above mentioned devices.

The spindle motor 205 may initiate or stop rotation of the microfluidic device 10 to allow the microfluidic device 10 to reach a specific position.

Although not shown, the spindle motor 205 may include a motor drive device to control an angular position of the microfluidic device 10. For example, the motor drive device may utilize a step motor or DC motor.

The data reading device 230 may be, e.g., a barcode reader. The data reading device 230 reads data stored in the barcode 13 and transmits the data to the controller 270. The controller 270 operates the respective devices based on the read data, to drive the sample testing apparatus.

The valve opening device 220 is provided to open or close the valves of the microfluidic device 10. The valve opening device 220 may include an external energy source 222 and moving units 224 and 226 to move the external energy source 222 to any one of the valves that needs to be opened or closed.

The external energy source 222 to radiate electromagnetic waves may be a laser light source to irradiate laser beam, or may be a light emitting diode or xenon lamp to irradiate visible or infrared light. In particular, the laser light source may include at least one laser diode.

The moving units 224 and 226 serve to regulate the position or orientation of the external energy source 222, so as to allow the external energy source 222 to focus energy to a desired region of the microfluidic device, i.e., to the valve to be opened or closed. The moving units 224 and 226 may include a drive motor 224 and a gear 226 to move the external energy source 222 mounted thereon to a position above the valve to be opened or closed via rotation of the drive motor 224. The moving units may be realized via various mechanisms.

The inspection device 240 may include at least one light emitting element 241 and a light receiving element 243 arranged to correspond to the light emitting element 241. Thus, the light receiving element 243 serves to receive light having passed through a reaction region of the microfluidic device 10.

The light emitting element 241 is a light source that can be turned on or off at a predetermined frequency. Exemplary light sources include, but are not limited to, semiconductor light emitting devices, such as Light Emitting Diodes (LEDs), Laser Diodes (LDs), etc., and gas discharge lamps, such as halogen lamps, xenon lamps, etc.

The light emitting element 241 is positioned to allow light emitted therefrom to reach the light receiving element 243 through the reaction region.

The light receiving element 243 is adapted to generate electric signals according to the intensity of incident light. For example, the light receiving element 243 may be a depletion layer photo diode, Avalanche Photo Diode (APD), PhotoMultiplier Tubes (PMT), or the like.

The controller 270 controls the spindle motor 205, data reading device 230, valve opening device 220, inspection device 240, etc., to assure effective operation of the sample testing apparatus. Also, the controller 270 searches the diagnosis DB 260 for comparative analysis between information detected from the inspection device 240 and the diagnosis DB 260, thereby testing for the presence of diseases of blood received in the reaction region on the microfluidic device 10.

The input device 210 serves to input the kind of the sample introduced into the microfluidic device 10 and/or possible testing items according to the kind of the injected sample, and may take the form of, for example, a touch screen provided at the sample testing apparatus.

The output device 250 serves to output the diagnosed results and the completion of operation. The output device 250 may be a visual output device, such as a Liquid Crystal Display (LCD), an audio output device, such as a speaker, or an audio-visual output device.

As is apparent from the above description, a metering chamber hinders leakage of a fluid until the chamber is full of the fluid, thereby preventing the metering chamber from being insufficiently filled with the fluid.

Further, the metering chamber may be filled with a preset accurate amount of fluid. Thus, an accurate amount of fluid may be used in a test, resulting in enhanced reliability in testing results of the fluid.

Although exemplary embodiments have been shown and described, it should be appreciated by those skilled in the art that changes may be made in these embodiments without departing from the principles and spirit of the inventive concept, the scope of which is defined in the claims and their equivalents.

## Claims

1. A microfluidic device comprising:
a platformcomprising:
a chamber configured to accommodate a fluid therein;
an inlet channel configured to supply the fluid into the chamber; and
an outlet channel connected to the chamber and provided separately from the inlet channel, wherein the outlet channel is configured such that after the chamber is filled with a predetermined amount of the fluid introduced through the inlet channel, any additional amount of the fluid introduced into the chamber in excess of the predetermined amount exits the chamber through the outlet channel.

2. The microfluidic device according to claim 1,
wherein the inlet channel includes a first inlet channel to guide the fluid toward the chamber and a second inlet channel to connect the first inlet channel to the chamber.

3. The microfluidic device according to claim 1,
wherein the outlet channel includes a first outlet channel to guide the fluid emerging from the chamber, and a second outlet channel to connect the first outlet channel to the chamber.

4. The microfluidic device according to claim 3,
wherein the second outlet channel is offset by a predetermined angle from a radial direction of the platform to a movement direction of the fluid in the first outlet channel, to ensure that the fluid emerging from the chamber moves into the first outlet channel.

5. The microfluidic device according to claim 1, wherein the chamber is a metering chamber configured to accommodate and discharge a predetermined amount of fluid.

6. The microfluidic device according to claim 1, further comprising a discharge channel connected to the chamber and configured to allow the fluid accommodated in the chamber to be discharged to the outside of the chamber.

7. The microfluidic device according to claim 6, further comprising a valve to open or close the discharge channel.

8. The microfluidic device according to claim 1, wherein centrifugal force is applied to the inlet channel and the outlet channel to enable movement of the fluid, and wherein the centrifugal force applied to the outlet channel is greater than the centrifugal force applied to the inlet channel.

9. The microfluidic device according to claim 1, wherein the chamber is located at an outer side of the inlet channel in a radial direction of the platform.

10. The microfluidic device according to claim 1, wherein the chamber is located at an outer side of the outlet channel in a radial direction of the platform.

11. The microfluidic device according to claim 1,
wherein a distance from a rotational center of the platform to the outlet channel is greater than a distance from the rotational center of the platform to the inlet channel such that centrifugal force applied to the outlet channel is greater than centrifugal force applied to the inlet channel during rotation of the platform.

12. A method of filling a chamber of a microfluidic device with a predetermined amount of fluid, the method comprising:
supplying a fluid into the chamber through an inlet channel of the microfluidic device connected to the chamber;
after the chamber is filled with a predetermined amount of the fluid through the inlet channel, allowing any additional amount of the fluid introduced to the chamber in excess of the predetermined amountto exit the chamber through an outlet channel of the microfluidic device that is connected to the chamber separately from the inlet channel; and
stopping the outflow of fluid through the outlet channel if the supplying of the fluid through the inlet channel is stopped, so as to allow the predetermined amount of the fluid to remain in the chamber.
